(19) **Europäisches Patentamt**
**European Patent Office**
**Office européen des brevets**

(11) **EP 4 398 257 A1**

(12) **EUROPEAN PATENT APPLICATION**
published in accordance with Art. 153(4) EPC

(43) Date of publication:
**10.07.2024 Bulletin 2024/28**

(21) Application number: **22864393.8**

(22) Date of filing: **25.08.2022**

(51) International Patent Classification (IPC):
***G16C 20/70*** (2019.01)

(52) Cooperative Patent Classification (CPC):
**G16C 20/70**

(86) International application number:
**PCT/JP2022/032048**

(87) International publication number:
**WO 2023/032809 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2021 JP 2021140833**

(71) Applicant: **Resonac Corporation
Tokyo 105-7325 (JP)**

(72) Inventors:
• **KAKUDA, Kohsuke
Tokyo 105-8518 (JP)**

• **MINAMI, Takuya
Tokyo 105-8518 (JP)**
• **AONUMA, Naoto
Tokyo 105-8518 (JP)**
• **TAKEMOTO, Shimpei
Tokyo 105-8518 (JP)**
• **TAKASHI, Hiroko
Tokyo 105-8518 (JP)**
• **OKUNO, Yoshishige
Tokyo 105-8518 (JP)**

(74) Representative: **Strehl Schübel-Hopf & Partner
Maximilianstrasse 54
80538 München (DE)**

(54) **PROPERTY PREDICTION DEVICE, PROPERTY PREDICTION METHOD, AND PROGRAM**

(57) A property prediction device for prediction of a property of a composite material composed of raw materials in a plurality of raw material categories. The property prediction device includes a prediction model creating part configured to create a prediction model by using a training dataset of a composite material including a raw material in a first raw material category and a raw material in a second raw material category to perform machine learning of a correspondence relationship between a property of the composite material, which is an objective variable, versus a mixing amount of the raw material in the first raw material category and a weighted feature of the raw material in the second raw material category, which are explanatory variables; and a prediction part configured to input, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

EP 4 398 257 A1

# FIG.3

PROPERTY PREDICTION DEVICE

30 — PREDICTION MODEL CREATING PART

50 — OBJECTIVE VARIABLE IDENTIFYING PART

52 — DESIGN CONDITION IDENTIFYING PART

54 — FEATURE CREATING PART

56 — EXPLANATORY VARIABLE CREATING PART

58 — LEARNING PROCESSING PART

32 — PREDICTION PART

34 — OUTPUT PART

40 — TRAINING DATASET STORAGE

42 — PREDICTION MODEL STORAGE

18

USER TERMINAL

26 — RESPONSE RECEIVING PART

24 — REQUEST TRANSMITTING PART

22 — OPERATION RECEIVING PART

20 — INFORMATION DISPLAY PART

**Description**

TECHNICAL FIELD

The present disclosure relates to a property prediction device, a property prediction method, and a program.

BACKGROUND ART

In recent years, properties of composite materials such as resin composite materials have been predicted by machine learning using computers.

Patent Document 1 describes a method for using training data including composition data and property data to perform a learning process on a model that outputs recommended composition data in response to target property data being input; and outputting a composition for producing a photosensitive resin composition having a target property in response to the target property being input into the model.

For example, Patent Document 1 describes that a composition indicated by composition data may be the presence or absence of a raw material from which a photosensitive resin composition can be produced, a compound included in the raw material (for example, the name or the structural formula of a specific compound included in the raw material indicated by a general name), or the content of the compound in the raw material.

Further, Patent Document 2 describes a method for using experimental values of physical properties of polymers and number densities of substructures of the polymers to construct a regression model that predicts a physical property value; and predicting the physical property value corresponding to an input polymer structure by using the regression model.

RELATED-ART DOCUMENTS

PATENT DOCUMENTS

Patent Document 1: Japanese Laid-open Patent Publication No. 2020-77346
Patent Document 2: Japanese Patent No. 6633820

SUMMARY OF THE INVENTION

PROBLEM TO BE SOLVED BY THE INVENTION

In a property prediction device for predicting a property of a composite material composed of raw materials in a plurality of raw material categories, a need exists for a technology by which, when a new raw material in a specific raw material category such as an additive is searched, the influence of the specific raw material category on the property of the composite material can be accurately predicted.

An object of the present disclosure is to provide a property prediction device, a property prediction method, and a program, in which the influence of a specific raw material category on a property of a composite material composed of raw materials in a plurality of raw material categories can be accurately predicted.

MEANS TO SOLVE THE PROBLEM

The present disclosure includes the following configurations.

[1] A property prediction device for prediction of a property of a composite material composed of raw materials in a plurality of raw material categories, the property prediction device including:

a prediction model creating part configured to create a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and
a prediction part configured to input, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

[2] The property prediction device according to [1], wherein the explanatory variables include information on mixing amounts of one or more raw materials included in the first raw material category, and information on weighted features that are products of features and mixing amounts of one or more raw materials included in the second raw material category.

[3] The property prediction device according to [1] or [2], wherein the prediction model creating part is configured to set, among the plurality of raw material categories of the composite material, a raw material category of a raw material that is searched for optimization as the second raw material category, and a raw material category other than the raw material category of the raw material that is searched for the optimization as the first raw material category.

[4] The property prediction device according to [3], wherein the prediction part is configured to predict the property of the composite material while searching a combination of one or more raw materials included in the second raw material category to be optimized and changing mixing amounts of the one or more raw materials included in the combination, and identify a combination of one or more raw materials and mixing amounts of the one or more raw materials included in the combination such that the predicted property approximates to a target property of the composite material corresponding to the prediction data.

[5] The property prediction device according to any one of [1] to [4], wherein the prediction model creating part includes

an objective variable identifying part configured to identify the property of the composite material as the objective variable based on the training dataset;

a design condition identifying part configured to identify design conditions of the composite material based on the training dataset,

a feature creating part configured to create features of one or more raw materials included in the second raw material category, and

an explanatory variable creating part configured to create, as the explanatory variables, mixing amounts of one or more raw materials included in the first raw material category and weighted features of the one or more raw materials included in the second raw material category, and

a learning processing part configured to create the prediction model by performing the machine learning of the correspondence relationship between the objective variable and the explanatory variables.

[6] The property prediction device according to any one of [1] to [5], wherein the composite material is a resin composite material including

a main raw material that is the raw material in the first raw material category, and

an additive that has a smaller mixing amount than a mixing amount of the main raw material and is the raw material in the second raw material category.

[7] The property prediction device according to any one of [1] to [6], wherein the plurality of raw material categories is a monomer, an oligomer, a polymer, a filler, a catalyst, a polymerization initiator, a polymerization inhibitor, a crosslinking agent, and a curing agent.

[8] The property prediction device according to any one of [1] to [7], wherein each of the features is information obtained by digitizing a structural characteristic of a molecule or information obtained by digitizing a chemical characteristic of the molecule.

[9] The property prediction device according to any one of [1] to [7], wherein each of the features is information obtained by describing, as a dummy variable represented by "0" or "1", a brand or a model number of each of the one or more raw materials included in the second raw material category.

[10] A property prediction method performed by a computer for predicting a property of a composite material composed of raw materials in a plurality of raw material categories, the property prediction method including:

a step of creating a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and

a step of inputting, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

[11] A program for causing a computer for predicting a property of a composite material composed of raw materials in a plurality of raw material categories, to execute a process including:

a step of creating a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and

a step of inputting, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

EFFECTS OF THE INVENTION

According to the present disclosure, the influence of a specific raw material category on a property of a composite material composed of raw materials in a plurality of raw material categories can be accurately predicted.

BRIEF DESCRIPTION OF THE DRAWINGS

[FIG. 1] FIG. 1 is a diagram illustrating an example of a configuration of an information processing system according to an embodiment;
[FIG. 2] FIG. 2 is a diagram illustrating an example of a hardware configuration of a computer according to the embodiment;
[FIG. 3] FIG. 3 is a diagram illustrating an example of a functional configuration of the information processing system according to the embodiment;
[FIG. 4] FIG. 4 is a diagram illustrating an example of a configuration of design conditions of resin composite materials;
[FIG. 5A] FIG. 5A is a diagram illustrating an example of a configuration of weighted features of a resin composite material;
[FIG. 5B] FIG. 5B is a diagram illustrating an example of a configuration of the weighted features of the resin composite material;
[FIG. 5C] FIG. 5C is a diagram illustrating an example of a configuration of the weighted features of the resin composite material;
[FIG. 6A] FIG. 6A is a diagram illustrating an example of a configuration of the mixing amounts of main raw materials and weighted features of additives of a resin composite material;
[FIG. 6B] FIG. 6B is a diagram illustrating an example of a configuration of the mixing amounts of the main raw materials and the weighted features of the additives of the resin composite material;
[FIG. 6C] FIG. 6C is a diagram illustrating an example of a configuration of the mixing amounts of the main raw materials and the weighted features of the additives of the resin composite material;
[FIG. 7] FIG. 7 is a diagram illustrating an example of an outline of a property prediction method performed by the information processing system according to the embodiment;
[FIG. 8] FIG. 8 is a diagram illustrating an example of a configuration of design conditions;
[FIG. 9A] FIG. 9A is a diagram illustrating an example of a process of creating weighted features of additives;
[FIG. 9B] FIG. 9B is a diagram illustrating the example of the process of creating the weighted features of the additives;
[FIG. 10] FIG. 10 is a diagram illustrating examples of created explanatory variables;
[FIG. 11] FIG. 11 is a flowchart illustrating an example of a process in a learning stage performed by the information processing system according to the embodiment;
[FIG. 12] FIG. 12 is an image diagram illustrating an example of a screen for selecting a material to be searched;
[FIG. 13] FIG. 13 is a diagram illustrating an example of a configuration of explanatory variables; and
[FIG. 14] FIG. 14 is a flowchart illustrating an example of a process in a prediction stage performed by the information processing system according to the embodiment.

MODE FOR CARRYING OUT THE INVENTION

Next, embodiments of the present invention will be described in detail. The present invention is not limited to the following embodiments. In the embodiments, a resin composite material will be described as an example of a composite material composed of raw materials in a plurality of raw material categories. The plurality of raw material categories is a monomer, an oligomer, a polymer, a filler, a catalyst, a polymerization initiator, a polymerization inhibitor, a crosslinking agent, a curing agent, and the like. The monomer, the oligomer, and the polymer are an example of a main raw material, and the filler, the catalyst, the polymerization initiator, the polymerization inhibitor, the crosslinking agent, and the curing agent are an example of an additive.

[First Embodiment]

<System Configuration>

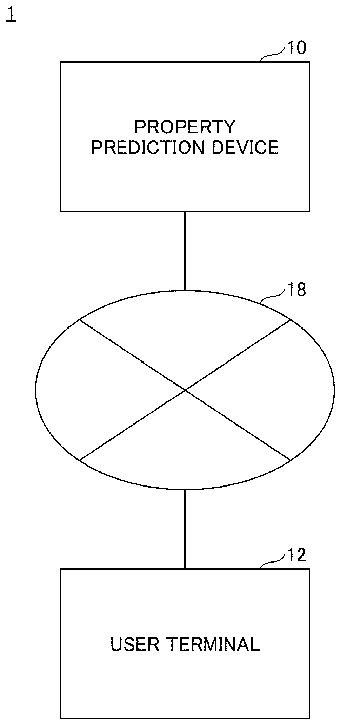

FIG. 1 is a diagram illustrating an example of a configuration of an information processing system according to an embodiment. An information processing system 1 of FIG. 1 includes a property prediction device 10 and a user terminal 12. The property prediction device 10 and the user terminal 12 are connected to each other via a communication network 18 such as a local area network (LAN) or the Internet so that data communication can be performed.
The user terminal 12 is an information processing terminal operated by a user, such as a PC, a tablet terminal, or a smartphone. The user terminal 12 receives, from the user, an input of information necessary to predict a property of a resin composite material composed of raw materials in a plurality of raw material categories, and causes the property prediction device 10 to predict the property of the resin composite material. Further, the user terminal 12 receives information on the property of the resin composite material predicted by the property prediction device 10, and displays the information on, for example, a display device to allow the user to confirm the information.
The property prediction device 10 is an information processing device, such as a PC or a workstation, that predicts the property of the resin composite material. The property prediction device 10 creates a prediction model by performing machine learning using a training dataset as will be described later. Upon receiving information necessary to predict the property of the resin composite material from the user terminal 12, the property prediction device 10 predicts the property of the resin composite material by using the prediction model. The property prediction device 10 transmits the predicted property of the resin composite material and other information to the user terminal 12.
The information processing system 1 of FIG. 1 is an example, and it is needless to say that there are various examples of system configurations according to applications and purposes. For example, the property prediction device 10 may be implemented by a plurality of computers or may be implemented as a cloud computing service. Further, the information processing system 1 of FIG. 1 may be implemented by a stand-alone computer.

<Hardware Configuration>

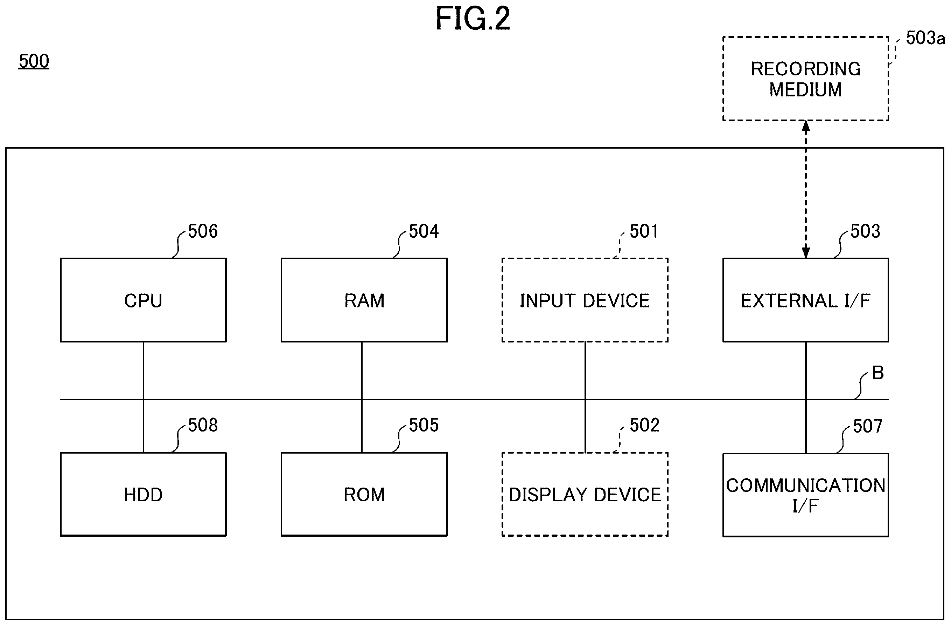

The property prediction device 10 and the user terminal 12 of FIG. 1 are implemented by, for example, a computer 500 having a hardware configuration illustrated in FIG. 2.
FIG. 2 is a diagram illustrating an example of a hardware configuration of the computer according to the present embodiment. The computer 500 of FIG. 2 includes an input device 501, a display device 502, an external I/F 503, a RAM 504, a ROM 505, a CPU 506, a communication I/F 507, an HDD 508, and the like, which are connected to each other via a bus B. Note that the input device 501 and the display device 502 may be configured to be connected and used.
The input device 501 is a touch panel, operation keys, buttons, a keyboard, a mouse, or the like used by the user to input various signals. The display device 502 includes a display such as a liquid crystal display or an organic EL display that displays a screen, a speaker that outputs sound data such as voice and sound, and the like. The communication I/F 507 is an interface for the computer 500 to perform data communication.
Further, the HDD 508 is an example of a non-volatile storage device that stores programs and data. The stored programs and data include an OS, which is basic software that controls the entire computer 500, applications that provide various functions on the OS, and the like. Note that the computer 500 may use a drive device (for example, a solid state drive (SSD)) using a flash memory as a storage media instead of the HDD 508.
The external I/F 503 is an interface with an external device. The external device includes a recording medium 503a and the like. This allows the computer 500 to read from and/or write to the recording medium 503a via the external I/F 503. The recording medium 503a includes a flexible disk, a CD, a DVD, an SD memory card, a USB memory, and the like.
The ROM 505 is an example of a non-volatile semiconductor memory (a storage device) that can retain programs and data even when the power is turned off. The ROM 505 stores programs and data such as a BIOS, which is executed when the computer 500 is started, OS settings, network settings, and the like. The RAM 504 is an example of a volatile semiconductor memory (a storage device) that temporarily stores programs and data.

The CPU 506 is an arithmetic device that reads programs and data from a storage device such as the ROM 505 or the HDD 508 onto the RAM 504 and executes a process to control the entire computer 500 and implement functions thereof. The computer 500 according to the present embodiment can implement various functions of the property prediction device 10 and the user terminal 12, as will be described later, by executing programs. The programs may be read from the recording medium 503a, in which the programs are stored, via the external I/F 503 and executed.

functional Configuration>

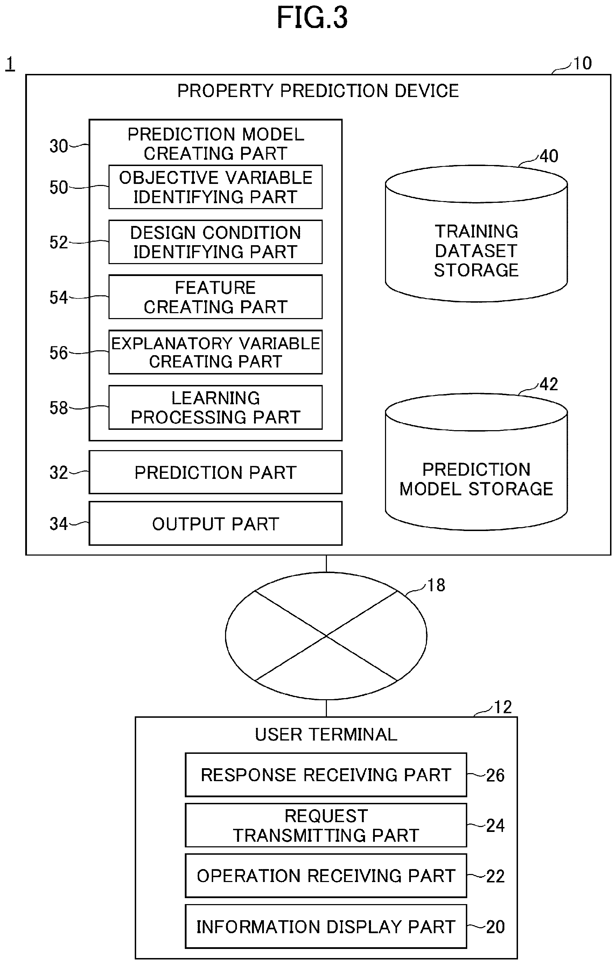

A configuration of the information processing system 1 according to the present embodiment will be described. FIG. 3 is a diagram illustrating an example of a functional configuration of the information processing system according to the present embodiment. Note that in the configuration diagram of FIG. 3, parts unnecessary for the description of the present embodiment are omitted as appropriate.

The property prediction device 10 of the information processing system 1 illustrated in FIG. 3 includes a prediction model creating part 30, a prediction part 32, an output part 34, a training dataset storage 40, and a prediction model storage 42. Further, the user terminal 12 includes an information display part 20, an operation receiving part 22, a request transmitting part 24, and a response receiving part 26.

The information display part 20 of the user terminal 12 displays information to be confirmed by the user on the display device 502. The operation receiving part 22 receives various operations from the user, such as an input of information necessary to predict a property of a resin composite material. The request transmitting part 24 transmits a request for processing such as predicting the property of the resin composite material, to the property prediction device 10. Further, the response receiving part 26 receives a response to the request for processing such as predicting the property of the resin composite material transmitted by the request transmitting part 24.

The training dataset storage 40 of the property prediction device 10 stores a training dataset of resin composite materials as will be described later. The prediction model creating part 30 creates a prediction model by performing machine learning using the training dataset stored in the training dataset storage 40, which will be described later. The prediction model storage 42 stores the created prediction model.

The prediction part 32 uses prediction data described later, which is information necessary to predict a property of a resin composite material and received from the user terminal 12, and the prediction model stored in the prediction model storage 42 to predict the property of the resin composite material corresponding to the prediction data. The output part 34 transmits the property of the resin composite material predicted by the prediction part 32, as a response to the request to predict the property of the resin composite material from the user terminal 12.

The prediction model creating part 30 of FIG. 3 includes an objective variable identifying part 50, a design condition identifying part 52, a feature creating part 54, an explanatory variable creating part 56, and a learning processing part 58. The objective variable identifying part 50 identifies a property of a resin composite material as an objective variable, based on the training dataset stored in the training dataset storage 40. The design condition identifying part 52 identifies design conditions of the resin composite material based on the training dataset stored in the training dataset storage 40.

The feature creating part 54 creates features of additives by identifying information on the additives based on the design conditions of the resin composite material. Each of the features is information obtained by digitizing a structural characteristic of a molecule or information obtained by digitizing a chemical characteristic of the molecule. The brands or the model numbers of the additives may be described as dummy variables represented by "0" and "1" and may be used as the features of the additives.

As an example of a method of digitizing a structural characteristic of a molecule, Extended Circular Fingerprints (hereinafter referred to as ECFPs) are used. The ECFPs digitizes the structural characteristic of the molecule by extracting the types and the numbers of all substructures and expressing them as vectors (column: the type, value: the number). The substructures can be expressed by a notation method such as Simplified Molecular Input Line Entry System (hereinafter referred to as SMILES) notation. The ECFPs can calculate the structural characteristic of the molecule, for example, by inputting information on an additive represented by the SMILES notation into an existing library. Further, as another example of a method of digitizing a structural characteristic of a molecule, a graph convolution neural network is used. The graph convolutional neural network can calculate the structural characteristic of the molecule, for example, by inputting information on an additive represented by the SMILES notation into an existing library.

Further, for example, as an example of a method of digitizing a chemical characteristic of a molecule, the chemical characteristic of the molecule is digitized by extracting physical property information of a molecule and representing it as a vector (column: the physical property information, value: a numerical number). The method of digitizing the chemical characteristic of the molecule can calculate the chemical characteristic of the molecule, for example, by inputting information on an additive represented by the SMILES notation into an existing library. The physical property information of the molecule is, for example, the molecular weight, the number of valence electrons, the partial charge, the number of amino groups, the number of hydroxyl groups, and the like. Further, the physical property information of the molecule may be a physical property value that can be calculated by quantum chemical calculation software, such as the HOMO, LUMO, electric charge, refractive index, or frequency, or a physical property value that can be measured by an experiment, such as the melting point, viscosity, or specific surface area.

The explanatory variable creating part 56 identifies information on main raw materials based on the design conditions of the resin composite material, and identifies the mixing amount of each of the main raw materials. Further, the explanatory variable creating part 56 calculates the products of the features of the additives, created by the feature creating part 54, and the mixing amounts of the additives. The products of the features of the additives and the mixing amounts of the additives are examples of weighted features. The explanatory variable creating part 56 creates explanatory variables by combining the mixing amounts of the main raw materials and the weighted features of the additives.

The learning processing part 58 creates a prediction model by machine learning of a correspondence relationship between the objective variable and the explanatory variables. The learning processing part 58 stores the created prediction model in the prediction model storage 42.

The prediction part 32 identifies design conditions of a resin composite material based on prediction data described later, which is information necessary to predict a property of the resin composite material and received from the user terminal 12. Similar to the explanatory variable creating part 56, the prediction part 32 creates explanatory variables based on the identified design conditions of the resin composite material, and inputs the explanatory variables into the prediction model so as to predict the property of the resin composite material corresponding to the prediction data.

Then, the output part 34 outputs the property of the resin composite material corresponding to the prediction data, predicted by the prediction part 32, as a response to a request to predict the property of the resin composite material from the user terminal 12. Note that the configuration diagram of FIG. 3 is an example. The configuration of the information processing system 1 according to the present embodiment may be considered in various ways.

<Study on Method for Predicting Property of Resin Composite Material>

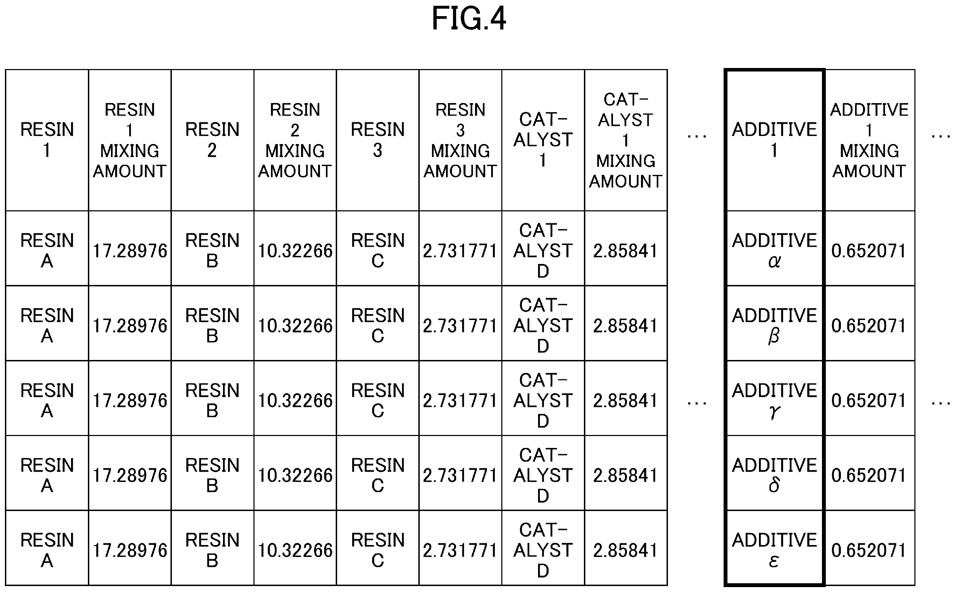

A property of a resin composite material composed of raw materials in a plurality of raw material categories can be predicted based on the names of a main raw material and an additive and the presence or absence thereof. FIG. 4 is a diagram illustrating an example of a configuration of design conditions of resin composite materials. In the design conditions as illustrated in FIG. 4, the mixing amounts are set for raw materials such as "resin 1", "resin 2", "resin 3", "catalyst 1", and "additive 1" included in each of the resin composite materials. It is assumed that in the design conditions of FIG. 4, "additive $\alpha$" to "additive $\varepsilon$" are unlearned additives. The brands or the model numbers of the main raw materials and of the additives, and the presence or absence thereof may be described as information that does not directly indicate physicochemical properties, for example, as dummy variables represented by "0" and "1".

In FIG. 4, design conditions other than the "additive 1" are the same. Therefore, even if the names of the additives are set to be different, namely "additive $\alpha$", "additive $\beta$", "additive $\gamma$", "additive $\delta$", and "additive $\varepsilon$", predicted values are less likely to differ, and thus the influence of the additives on the property of the resin composite material would not be predicted with high accuracy.

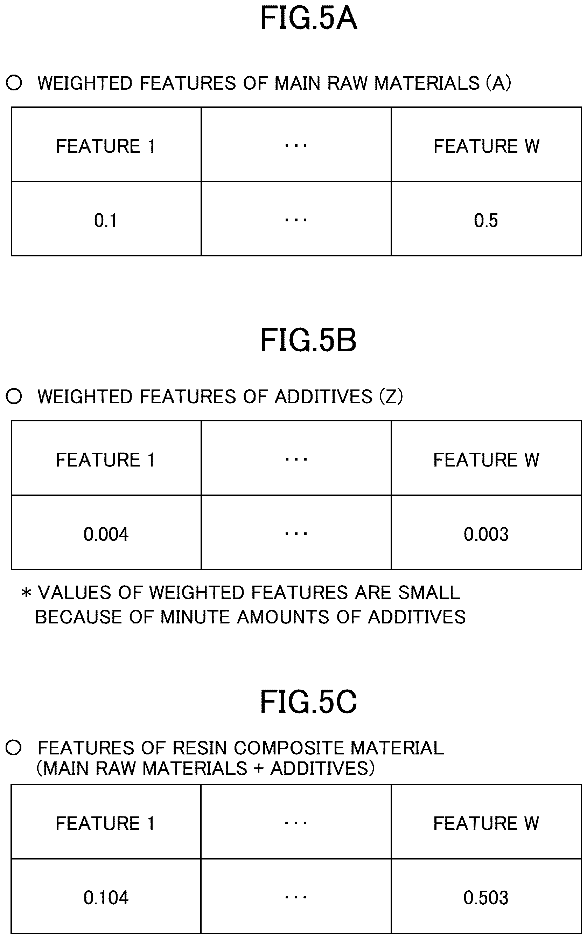

Further, a property of a resin composite material composed of raw materials in a plurality of raw material categories can be predicted based on weighted features of a main raw material and an additive. FIG. 5A through FIG. 5C are diagrams illustrating examples of configurations of weighted features of a resin composite material. A weighted feature is information obtained by weighting a feature of each of a main raw material and an additive by the mixing amount (or the mixing ratio) of each of the main raw material and the additive. The whole mixing ratio (100%) is the total amount of mixed raw materials. A feature of the resin composite material is calculated as the sum of weighted features of all the raw materials.

In the examples of FIG. 5A through FIG. 5C, the mixing amounts of additives (Z) are smaller than the mixing amounts of main raw materials (A), and thus weighted features of the additives (Z) are smaller than weighted features of the main raw materials (A). Because the weighted features of the additives (Z) are smaller than the weighted features of the main raw materials (A), if some substructures of the main raw materials (A) and the additives (Z) are the same, changes in the weighted features of the additives (Z) may be hidden. Therefore, the changes in the weighted features of the additives (Z) are unlikely to be reflected in features of the resin composite material, and the influence of the additives (Z) on the property of the resin composite material would not be predicted with high accuracy.

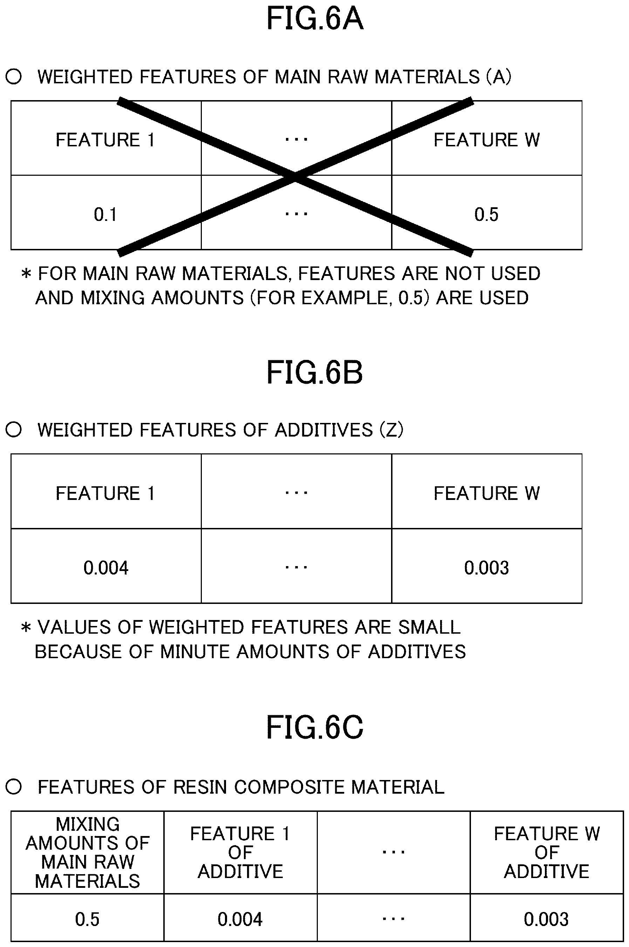

In view of the above, in the present embodiment, a property of a resin composite material composed of raw materials in a plurality of raw material categories is predicted based on the mixing amounts of main raw materials and weighted features of additives. FIG. 6A through FIG. 6C are diagrams illustrating examples of configurations of the mixing amounts of the main raw materials and the weighted features of the additives of a resin composite material. In the example of FIG. 6A through FIG. 6C, features of the main raw materials are not used and the mixing amounts of the main raw materials are used.

Accordingly, in the examples of FIG. 6A through FIG. 6C, even if some substructures of main raw materials (A) and additives (Z) are the same, changes in the weighted features of the additives (Z) are not hidden in the weighted features of the main raw materials (A). Therefore, the changes in the weighted features of the additives (Z) are easily reflected in features of the resin composite material, and the influence of the additives (Z) on the property of the resin composite material can be predicted with high accuracy.

<Process>

A process for predicting a property of a resin composite material composed of raw materials in a plurality of raw material categories, performed by the information processing system 1 according to the present embodiment, will be described.

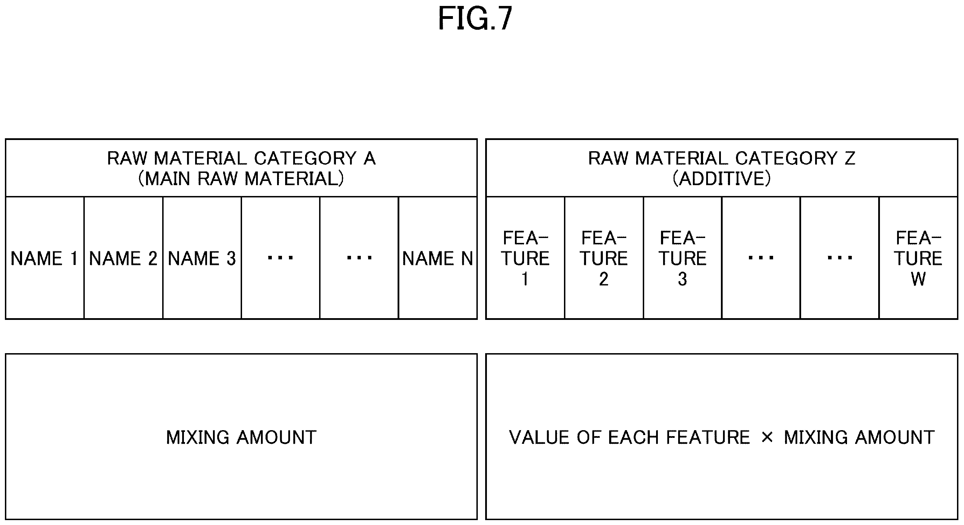

FIG. 7 is a diagram illustrating an example of an outline of a property prediction method performed by the information processing system according to the present embodiment. FIG. 7 illustrates an example in which explanatory variables for predicting a property of a resin composite material composed of raw materials in a plurality of raw material categories is created by combining columns (N-dimension) in which the names of N types of raw materials in a raw material category A that is a main raw material are used as variables, with columns (W-dimension) of weighted features of raw materials in a raw material category Z that is an additive.

The example of FIG. 7 illustrates explanatory variables in a case were additives in the raw material category Z are searched. It is conceivable that the example of FIG. 7 illustrates explanatory variables in a case were main raw materials in the raw material category A are not searched. The explanatory variables illustrated in FIG. 7 are created based on the mixing amounts of the N types of raw materials in the raw material category A that is the main raw material, and weighted features (values of features × mixing amounts) of W types of raw materials in the raw material category Z that is the additive. The mixing amounts can be expressed as molar ratios, or may be expressed as weight ratios.

In the present embodiment, the explanatory variables as illustrated in FIG. 7 are used to create a prediction model that can predict the property of the resin composite material. Specifically, a prediction model is created by using the training dataset to perform machine learning of a correspondence relationship between the property of the resin composite material, which is an objective variable, and the explanatory variables as illustrated in FIG. 7.

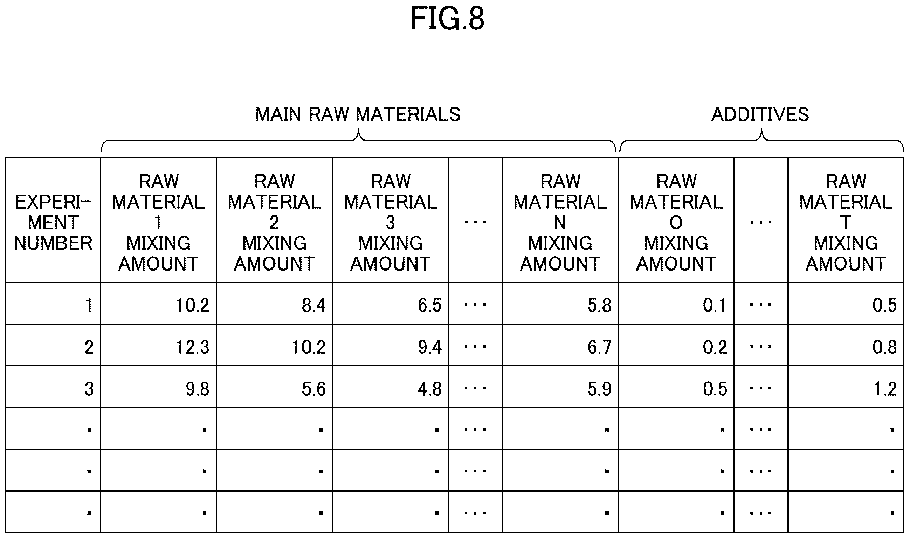

FIG. 8 is a diagram illustrating an example of design conditions. In the example illustrated FIG. 8, the design conditions include, as information, the mixing amounts of N types of raw materials, which are main raw materials, and the mixing amounts of "raw material O" to "raw material T", which are additives. The mixing amount of each of the main raw materials is used as an explanatory variable element as indicated in the following formula (1).

[Formula 1]

$$x_{i,j} : \text{MIXING AMOUNT} \quad \cdots(1)$$

$i$ : EXPERIMENT NUMBER
$j$ : RAW MATERIAL NUMBER, $1 \le j \le N$

For each of the additives, a weighted feature calculated as indicated by the following formula (2) is used as an explanatory variable element.

[Formula 2]

$$x_{i,w} = \sum_{j} \text{MIXING AMOUNT}_{i,j} \times \text{FEATURE}_{j,w} \quad \cdots(2)$$

$i$ : EXPERIMENT NUMBER
$j$ : RAW MATERIAL NUMBER, $0 \le j \le T$
$w$ : FEATURE NUMBER, $1 \le w \le W$

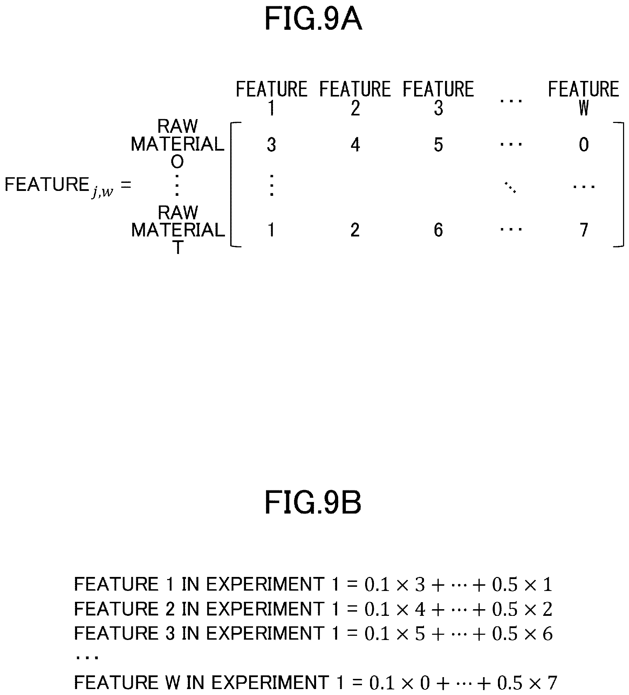

FIG. 9A and FIG. 9B are diagrams illustrating an example of a process of creating weighted features of additives. As illustrated in FIG. 9A, a feature can be represented by a matrix of j (an additive) × w (a feature) for each experiment number i. Weighted features can be calculated by the formula (2) as illustrated in FIG. 9B, based on the mixing amounts of the "raw material O" to the "raw material T" illustrated in FIG. 8, which are the additives, and "feature 1" to "feature W" illustrated in FIG. 9A.

For example, FIG. 9B illustrates an example in which weighted features of the additives are calculated based on design conditions of "experiment 1" having an experiment number of "1". In the example of FIG. 9B, weighted features are calculated with respect to the "feature 1" to the "feature W". The "feature 1 in experiment 1" of FIG. 9B is calculated by obtaining the sum of weighted features, which are the products of the mixing amounts "0.1" to "0.5" of the "raw material O" to the "raw material T" in the "experiment 1" of FIG. 8 and features "3" to "1" of the "raw material O" to the "raw material T" of FIG. 9A.

Further, the "feature 2 in experiment 1" to the "feature W in experiment 1" of FIG. 9B are calculated in the same manner as the "feature 1 in experiment 1". Although not illustrated, weighted features of the additives are calculated in the same manner as the "experiment 1", based on design conditions of experiments having an experiment number of "2" and the subsequent numbers of FIG. 8.

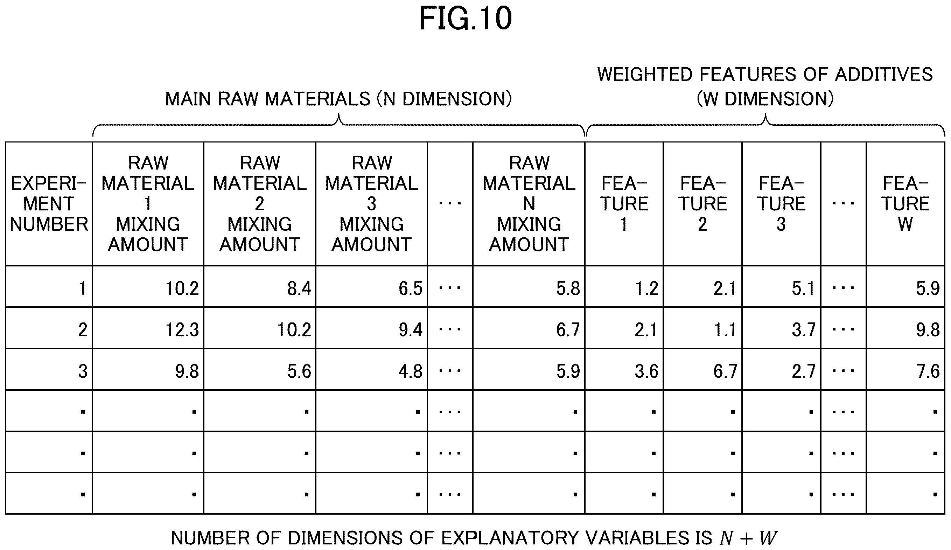

Explanatory variables as illustrated in FIG. 10 can be created by combining columns of the mixing amounts of the main raw materials (N dimension) and columns of the weighted features (W dimension) of the additives. FIG. 10 is a diagram illustrating examples of the created explanatory variables. As illustrated in FIG. 10, the number of dimensions of the explanatory variables is N + W.

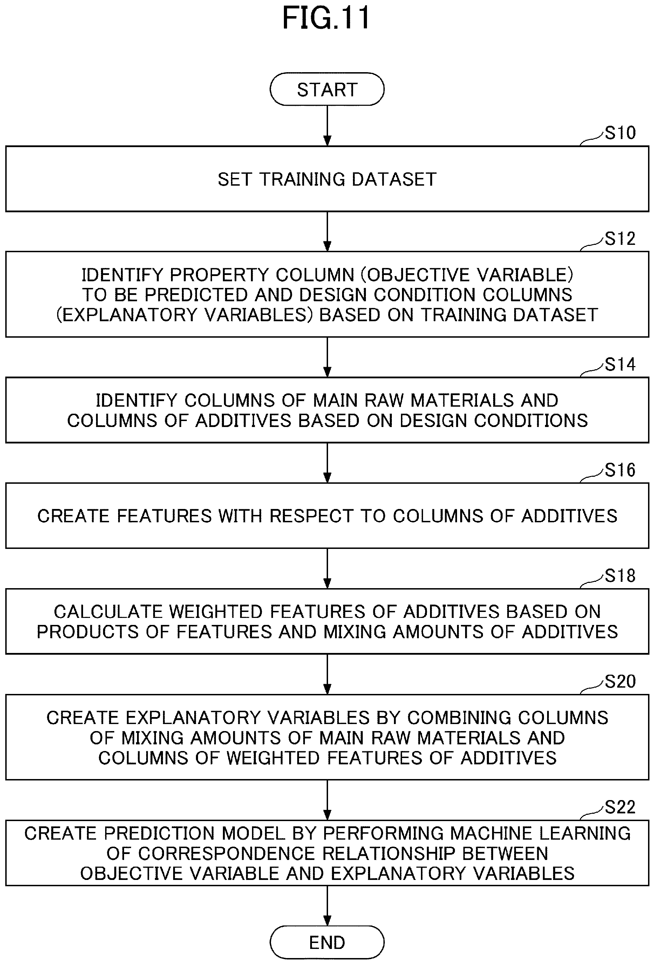

FIG. 11 is a flowchart illustrating an example of a process in a learning stage performed by the information processing system according to the embodiment. In step S10, the prediction model creating part 30 of the property prediction device 10 receives a training dataset to be set. In step S10, a training dataset may be selected from one or more training datasets stored in the training dataset storage 40 and may be set. Further, receiving the training dataset to be set in step S10 may be a process of storing one training dataset, received from the user, in the training dataset storage 40.

In step S12, the objective variable identifying part 50 of the prediction model creating part 30 identifies a property column (an objective variable) to be predicted based on the training dataset received in step S10. Further, the design condition identifying part 52 identifies design condition columns (explanatory variables) as illustrated in FIG. 8 based on the training dataset received in step S10.

In step S14, the explanatory variable creating part 56 identifies columns of main raw materials based on the design condition columns identified in step S12. Further, the feature creating part 54 identifies columns of additives based on the design condition columns identified in step S12. In step S16, the feature creating part 54 creates features as illustrated in, for example, FIG. 9A with respect to the columns of the additives identified in step S14.

In step S18, the explanatory variable creating part 56 calculates weighted features of the additives as illustrated in FIG. 9B, based on the products of the features of the additives, created by the feature creating part 54 in step S16 as illustrated in FIG. 9A, and the mixing amounts of the additives as illustrated in FIG. 8.

In step S20, the explanatory variable creating part 56 creates explanatory variables as illustrated in FIG. 10, by combining the columns of the mixing amounts of the main raw materials identified in step S14 and the columns of the weighted features of the additives calculated in step S18.

In step S22, the learning processing part 58 creates a prediction model by performing machine learning of a correspondence relationship between the objective variable identified in step S12 and the explanatory variables created in step S20. The created prediction model is stored in the prediction model storage 42.

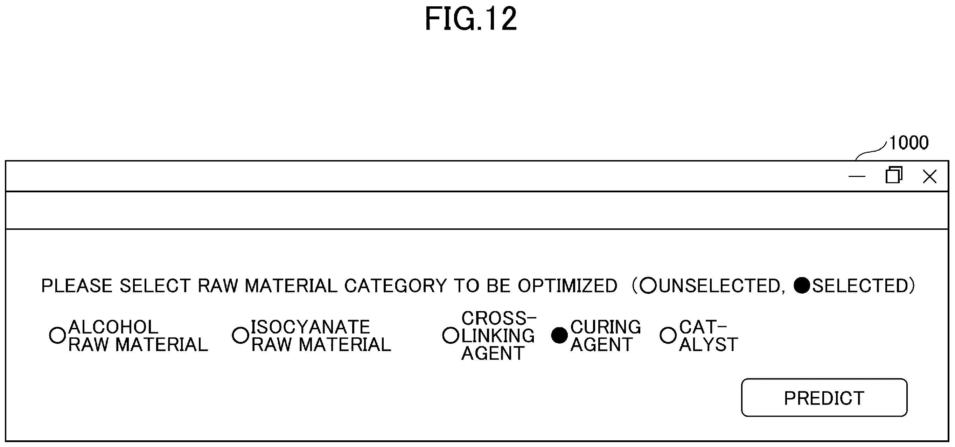

Although an example in which the additives are searched has been described above, a raw material to be searched may be selected from a screen 1000 as illustrated in FIG. 12, for example. FIG. 12 is an image diagram illustrating an example of a screen for selecting a material to be searched.

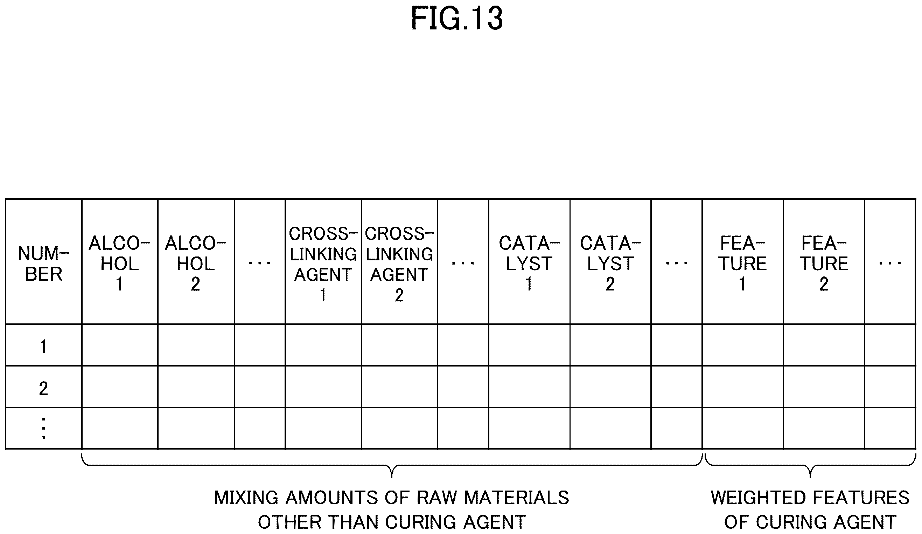

The screen 1000 of FIG. 12 is a graphical user interface (GUI) for selecting, as a raw material category to be optimized, a raw material category to be searched from among a plurality of raw material categories of a resin composite material. The user selects a raw material category to be optimized, from among raw material categories such as an alcohol raw material, an isocyanate raw material, a crosslinking agent, a curing agent, and a catalyst displayed on the screen 1000. For example, in FIG. 12, the "curing agent" is selected as a raw material category to be optimized. In the state illustrated FIG. 12, upon a "predict" button being pressed, the prediction model creating part 30 of the property prediction device 10 creates explanatory variables as illustrated in FIG. 13 in accordance with the flowchart illustrated in FIG. 11, for example.

FIG. 13 is a diagram illustrating an example of a configuration of explanatory variables. The explanatory variables of FIG. 13 are created by combining weighted features in the "curing agent" category selected as the raw material category to be optimized on the screen 1000 of FIG. 12, with the mixing amounts in raw material categories not selected as the raw material category to be optimized. As illustrated in FIG. 13, the information processing system 1 according to the present embodiment can create the explanatory variables by combining elements represented as the mixing amounts of raw materials in the raw material categories not selected as the raw material category to be optimized, with elements represented as weighted features of raw materials of the raw material category selected as the raw material category to be optimized, and can use the created explanatory variables for machine learning.

In the present embodiment, an example in which a raw material category to be optimized is the additive category has been described. However, as indicated by the screen 1000 of FIG. 12, a raw material category to be optimized may be the main raw material category. For example, if the "isocyanate raw material" category is selected as a raw material category to be optimized on the screen 1000 of FIG. 12, explanatory variables are created by combining weighted features in isocyanate raw materials in the "isocyanate raw material" category, with the mixing amounts of alcohol raw materials, crosslinking agents, curing agents, and catalysts. Further, although one raw material category to be optimized is selected on the screen 1000 of FIG. 12, a plurality of raw material categories may be selected.

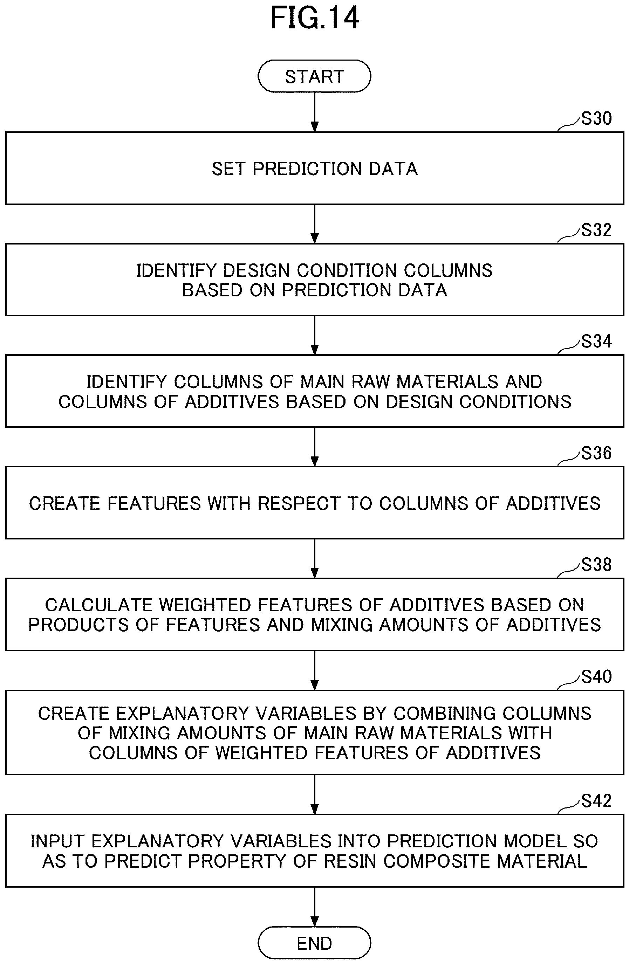

FIG. 14 is a flowchart illustrating an example of a process in a prediction stage performed by the information processing system according to the present embodiment. In step S30, the prediction part 32 of the property prediction device 10 receives prediction data to be set. In step S32, the prediction part 32 identifies design condition columns as illustrated in, for example, FIG. 8, based on the prediction data received in step S30.

In step S34, the prediction part 32 identifies columns of main raw materials and columns of additives based on the design condition columns identified in step S32. In step S36, the prediction part 32 creates features as illustrated in, for example, FIG. 9A with respect to the columns of the additives identified in step S34.

In step S38, the prediction part 32 calculates weighted features of the additives as illustrated in FIG. 9B, based on the products of the features of the additives, created in step S36 as illustrated in FIG. 9A, and the mixing amounts of the additives as illustrated in FIG. 8.

In step S40, the prediction part 32 creates explanatory variables as illustrated in FIG. 10, by combining the columns of the mixing amounts of the main raw materials identified in step S34 with the columns of the weighted features of the additives calculated in step S38. In step S42, the prediction part 32 inputs the explanatory variables created in step S40 into a prediction model so as to predict a property of a resin composite material corresponding to the prediction data.

The process of the flowchart of FIG. 14 is repeated while searching a combination of raw materials (additives) and changing the mixing amounts of the raw materials included in the combination, until the predicted property of the resin composite material achieves the target property of the resin composite material or until the set number of times (for example, 10,000 times) of calculation is completed. The prediction part 32 predicts the property of the resin composite material by using a combination of raw materials (additives) and the mixing amounts of the raw materials included in the combination as exhaustive search points.

For example, exhaustive search points can be generated by generating mixing amounts of raw materials included in a combination at random within a predetermined range or at predetermined intervals. By using exhaustive search points at which the predicted property of the resin composite material approximates to the target property, the prediction part 32 can identify a combination of raw materials (additives) and the mixing amounts of the raw materials included in the combination such that the predicted property approximates to the target property of the resin composite material.

[Other Embodiments]

The property of the resin composite material predicted by the property prediction device 10 according to the present embodiment can be applied to a mix design device or a mix design support device used for a composite material composed of raw materials in a plurality of raw material categories, a program for implementing the mix design device or the mix design support device, and the like. Further, the property prediction device 10 according to the present embodiment may cause a manufacturing device to produce a resin composite material by supplying design conditions under which a predicted property is close to a target property, to the manufacturing apparatus.

As described above, with respect to a composite material composed of raw materials in a plurality of raw material categories, the information processing system 1 of the present embodiment can create explanatory variables by calculating weighted features of raw materials in a specific raw material category, and combining the weighted features with the mixing amounts of raw materials in another raw material category. Accordingly, the influence of the other material category can be reduced, and the influence of the specific raw material category on the property of the composite material can be accurately predicted.

For example, the information processing system 1 according to the present embodiment can accurately predict a change in a property of a composite material when the molecular structure of an additive (addition) having a smaller mixing amount than that of a main raw material is changed. In addition, in order to satisfy a desired property, additives to be used can be screened. The information processing system 1 according to the present embodiment is particularly effective when some of substructures of an additive and a main raw material are the same.

Although the embodiments have been described above, various changes in form and detail may be made without departing from the spirit and scope of the appended claims. The present invention has been described based on the embodiments; however, the present invention is not limited to the above-described embodiments, and various modifications can be made within the scope described in the claims. This application is based on and claims priority to Japanese Patent Application No. 2021-140833, filed on August 31, 2021, the entire contents of which are incorporated herein by reference.

DESCRIPTION OF THE REFERENCE NUMERALS

| | |
|---|---|
| 1 | information processing system |
| 10 | property prediction device |
| 12 | user terminal |
| 18 | communication network |
| 20 | information display part |
| 22 | operation receiving part |
| 24 | request transmitting part |
| 26 | response receiving part |
| 30 | prediction model creating part |
| 32 | prediction part |
| 34 | output part |
| 40 | training dataset storage |
| 42 | prediction model storage |
| 50 | objective variable identifying part |
| 52 | design condition identifying part |
| 54 | feature creating part |
| 56 | explanatory variable creating part |
| 58 | learning processing part |

**Claims**

1. A property prediction device for prediction of a property of a composite material composed of raw materials in a plurality of raw material categories, the property prediction device comprising:

   a prediction model creating part configured to create a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and
   a prediction part configured to input, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

2. The property prediction device according to claim 1, wherein the explanatory variables include information on mixing amounts of one or more raw materials included in the first raw material category, and information on weighted features that are products of features and mixing amounts of one or more raw materials included in the second raw material category.

3. The property prediction device according to claim 1 or 2, wherein the prediction model creating part is configured to set, among the plurality of raw material categories of the composite material, a raw material category of a raw material that is searched for optimization as the second raw material category, and a raw material category other than the raw material category of the raw material that is searched for the optimization as the first raw material category.

4. The property prediction device according to claim 3, wherein the prediction part is configured to predict the property of the composite material while searching a combination of one or more raw materials included in the second raw material category to be optimized and changing mixing amounts of the one or more raw materials included in the combination, and identify a combination of one or more raw materials and mixing amounts of the one or more raw materials included in the combination such that the predicted property approximates to a target property of the composite material corresponding to the prediction data.

**5.** The property prediction device according to any one of claims 1 to 4, wherein the prediction model creating part includes

an objective variable identifying part configured to identify the property of the composite material as the objective variable based on the training dataset;

a design condition identifying part configured to identify design conditions of the composite material based on the training dataset,

a feature creating part configured to create features of one or more raw materials included in the second raw material category, and

an explanatory variable creating part configured to create, as the explanatory variables, mixing amounts of one or more raw materials included in the first raw material category and weighted features of the one or more raw materials included in the second raw material category, and

a learning processing part configured to create the prediction model by performing the machine learning of the correspondence relationship between the objective variable and the explanatory variables.

**6.** The property prediction device according to any one of claims 1 to 5, wherein the composite material is a resin composite material including

a main raw material that is the raw material in the first raw material category, and

an additive that has a smaller mixing amount than a mixing amount of the main raw material and is the raw material in the second raw material category.

**7.** The property prediction device according to any one of claims 1 to 6, wherein the plurality of raw material categories is a monomer, an oligomer, a polymer, a filler, a catalyst, a polymerization initiator, a polymerization inhibitor, a crosslinking agent, and a curing agent.

**8.** The property prediction device according to any one of claims 1 to 7, wherein each of the features is information obtained by digitizing a structural characteristic of a molecule or information obtained by digitizing a chemical characteristic of the molecule.

**9.** The property prediction device according to any one of claims 1 to 7, wherein each of the features is information obtained by describing, as a dummy variable represented by "0" or "1", a brand or a model number of each of the one or more raw materials included in the second raw material category.

**10.** A property prediction method performed by a computer for predicting a property of a composite material composed of raw materials in a plurality of raw material categories, the property prediction method comprising:

a step of creating a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and

a step of inputting, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

**11.** A program for causing a computer for predicting a property of a composite material composed of raw materials in a plurality of raw material categories, to execute a process comprising:

a step of creating a prediction model by using a training dataset of a composite material including a raw material in a first raw material category and a raw material in a second raw material category to perform machine learning of a correspondence relationship between a property of the composite material versus a mixing amount of the raw material in the first raw material category and a weighted feature of the raw material in the second raw material category, the property of the composite material being an objective variable, and the mixing amount and the weighted feature being explanatory variables; and

a step of inputting, as explanatory variables, a mixing amount of a raw material in the first raw material category and a weighted feature of a raw material in the second raw material category, that are created based on prediction data of a composite material whose property is to be predicted, into the prediction model so as to predict the property of the composite material corresponding to the prediction data.

1

PROPERTY
PREDICTION DEVICE
10

18

USER TERMINAL
12

# FIG.2

# FIG.3

1                                                            10

**PROPERTY PREDICTION DEVICE**

30 — PREDICTION MODEL CREATING PART

50 — OBJECTIVE VARIABLE IDENTIFYING PART

52 — DESIGN CONDITION IDENTIFYING PART

54 — FEATURE CREATING PART

56 — EXPLANATORY VARIABLE CREATING PART

58 — LEARNING PROCESSING PART

40 — TRAINING DATASET STORAGE

32 — PREDICTION PART

42 — PREDICTION MODEL STORAGE

34 — OUTPUT PART

18

**USER TERMINAL** — 12

RESPONSE RECEIVING PART — 26

REQUEST TRANSMITTING PART — 24

OPERATION RECEIVING PART — 22

INFORMATION DISPLAY PART — 20

# FIG.4

| RESIN 1 | RESIN 1 MIXING AMOUNT | RESIN 2 | RESIN 2 MIXING AMOUNT | RESIN 3 | RESIN 3 MIXING AMOUNT | CAT-ALYST 1 | CAT-ALYST 1 MIXING AMOUNT | ... | ADDITIVE 1 | ADDITIVE 1 MIXING AMOUNT | ... |
|---|---|---|---|---|---|---|---|---|---|---|---|
| RESIN A | 17.28976 | RESIN B | 10.32266 | RESIN C | 2.731771 | CAT-ALYST D | 2.85841 | | ADDITIVE $\alpha$ | 0.652071 | |
| RESIN A | 17.28976 | RESIN B | 10.32266 | RESIN C | 2.731771 | CAT-ALYST D | 2.85841 | | ADDITIVE $\beta$ | 0.652071 | |
| RESIN A | 17.28976 | RESIN B | 10.32266 | RESIN C | 2.731771 | CAT-ALYST D | 2.85841 | ... | ADDITIVE $\gamma$ | 0.652071 | ... |
| RESIN A | 17.28976 | RESIN B | 10.32266 | RESIN C | 2.731771 | CAT-ALYST D | 2.85841 | | ADDITIVE $\delta$ | 0.652071 | |
| RESIN A | 17.28976 | RESIN B | 10.32266 | RESIN C | 2.731771 | CAT-ALYST D | 2.85841 | | ADDITIVE $\varepsilon$ | 0.652071 | |

EP 4 398 257 A1

# FIG.5A

○ WEIGHTED FEATURES OF MAIN RAW MATERIALS (A)

| FEATURE 1 | · · · | FEATURE W |
|---|---|---|
| 0.1 | · · · | 0.5 |

# FIG.5B

○ WEIGHTED FEATURES OF ADDITIVES (Z)

| FEATURE 1 | · · · | FEATURE W |
|---|---|---|
| 0.004 | · · · | 0.003 |

＊ VALUES OF WEIGHTED FEATURES ARE SMALL
BECAUSE OF MINUTE AMOUNTS OF ADDITIVES

# FIG.5C

○ FEATURES OF RESIN COMPOSITE MATERIAL
(MAIN RAW MATERIALS + ADDITIVES)

| FEATURE 1 | · · · | FEATURE W |
|---|---|---|
| 0.104 | · · · | 0.503 |

# FIG.6A

○ WEIGHTED FEATURES OF MAIN RAW MATERIALS (A)

| FEATURE 1 | · · · | FEATURE W |
|-----------|-------|-----------|
| 0.1 | · · · | 0.5 |

* FOR MAIN RAW MATERIALS, FEATURES ARE NOT USED
AND MIXING AMOUNTS (FOR EXAMPLE, 0.5) ARE USED

# FIG.6B

○ WEIGHTED FEATURES OF ADDITIVES (Z)

| FEATURE 1 | · · · | FEATURE W |
|-----------|-------|-----------|
| 0.004 | · · · | 0.003 |

* VALUES OF WEIGHTED FEATURES ARE SMALL
BECAUSE OF MINUTE AMOUNTS OF ADDITIVES

# FIG.6C

○ FEATURES OF RESIN COMPOSITE MATERIAL

| MIXING AMOUNTS OF MAIN RAW MATERIALS | FEATURE 1 OF ADDITIVE | · · · | FEATURE W OF ADDITIVE |
|---------------------------------------|-----------------------|-------|-----------------------|
| 0.5 | 0.004 | · · · | 0.003 |

# FIG.7

| RAW MATERIAL CATEGORY A (MAIN RAW MATERIAL) | | | | | |
|---|---|---|---|---|---|
| NAME 1 | NAME 2 | NAME 3 | · · · | · · · | NAME N |

| RAW MATERIAL CATEGORY Z (ADDITIVE) | | | | | |
|---|---|---|---|---|---|
| FEA-TURE 1 | FEA-TURE 2 | FEA-TURE 3 | · · · | · · · | FEA-TURE W |

| MIXING AMOUNT | VALUE OF EACH FEATURE × MIXING AMOUNT |
|---|---|

EP 4 398 257 A1

# FIG.8

| EXPERI-MENT NUMBER | MAIN RAW MATERIALS | | | | | ADDITIVES | | |
|---|---|---|---|---|---|---|---|---|
| | RAW MATERIAL 1 MIXING AMOUNT | RAW MATERIAL 2 MIXING AMOUNT | RAW MATERIAL 3 MIXING AMOUNT | ··· | RAW MATERIAL N MIXING AMOUNT | RAW MATERIAL O MIXING AMOUNT | ··· | RAW MATERIAL T MIXING AMOUNT |
| 1 | 10.2 | 8.4 | 6.5 | ··· | 5.8 | 0.1 | ··· | 0.5 |
| 2 | 12.3 | 10.2 | 9.4 | ··· | 6.7 | 0.2 | ··· | 0.8 |
| 3 | 9.8 | 5.6 | 4.8 | ··· | 5.9 | 0.5 | ··· | 1.2 |
| . | . | . | . | ··· | . | . | ··· | . |
| . | . | . | . | ··· | . | . | ··· | . |
| . | . | . | . | ··· | . | . | ··· | . |

EP 4 398 257 A1

## FIG.9A

$$\text{FEATURE}_{j,w} = \begin{array}{c} \text{RAW} \\ \text{MATERIAL} \\ \text{O} \\ \vdots \\ \text{RAW} \\ \text{MATERIAL} \\ \text{T} \end{array}
\begin{array}{ccccc}
\text{FEATURE} & \text{FEATURE} & \text{FEATURE} & & \text{FEATURE} \\
1 & 2 & 3 & \cdots & \text{W} \\
\end{array}
\left[\begin{array}{ccccc}
3 & 4 & 5 & \cdots & 0 \\
\vdots & & & \ddots & \cdots \\
1 & 2 & 6 & \cdots & 7 \\
\end{array}\right]$$

## FIG.9B

FEATURE 1 IN EXPERIMENT 1 $= 0.1 \times 3 + \cdots + 0.5 \times 1$

FEATURE 2 IN EXPERIMENT 1 $= 0.1 \times 4 + \cdots + 0.5 \times 2$

FEATURE 3 IN EXPERIMENT 1 $= 0.1 \times 5 + \cdots + 0.5 \times 6$

...

FEATURE W IN EXPERIMENT 1 $= 0.1 \times 0 + \cdots + 0.5 \times 7$

# FIG.10

|  | MAIN RAW MATERIALS (N DIMENSION) | | | | | WEIGHTED FEATURES OF ADDITIVES (W DIMENSION) | | | | |
|---|---|---|---|---|---|---|---|---|---|---|
| EXPERI-MENT NUMBER | RAW MATERIAL 1 MIXING AMOUNT | RAW MATERIAL 2 MIXING AMOUNT | RAW MATERIAL 3 MIXING AMOUNT | ··· | RAW MATERIAL N MIXING AMOUNT | FEA-TURE 1 | FEA-TURE 2 | FEA-TURE 3 | ··· | FEA-TURE W |
| 1 | 10.2 | 8.4 | 6.5 | ··· | 5.8 | 1.2 | 2.1 | 5.1 | ··· | 5.9 |
| 2 | 12.3 | 10.2 | 9.4 | ··· | 6.7 | 2.1 | 1.1 | 3.7 | ··· | 9.8 |
| 3 | 9.8 | 5.6 | 4.8 | ··· | 5.9 | 3.6 | 6.7 | 2.7 | ··· | 7.6 |
| . | . | . | . | ··· | . | . | . | . | ··· | . |
| . | . | . | . | ··· | . | . | . | . | ··· | . |
| . | . | . | . | ··· | . | . | . | . | ··· | . |

NUMBER OF DIMENSIONS OF EXPLANATORY VARIABLES IS $N + W$

EP 4 398 257 A1

# FIG.11

START

S10

SET TRAINING DATASET

S12

IDENTIFY PROPERTY COLUMN (OBJECTIVE VARIABLE)
TO BE PREDICTED AND DESIGN CONDITION COLUMNS
(EXPLANATORY VARIABLES) BASED ON TRAINING DATASET

S14

IDENTIFY COLUMNS OF MAIN RAW MATERIALS AND
COLUMNS OF ADDITIVES BASED ON DESIGN CONDITIONS

S16

CREATE FEATURES WITH RESPECT TO COLUMNS OF ADDITIVES

S18

CALCULATE WEIGHTED FEATURES OF ADDITIVES BASED ON
PRODUCTS OF FEATURES AND MIXING AMOUNTS OF ADDITIVES

S20

CREATE EXPLANATORY VARIABLES BY COMBINING COLUMNS
OF MIXING AMOUNTS OF MAIN RAW MATERIALS AND
COLUMNS OF WEIGHTED FEATURES OF ADDITIVES

S22

CREATE PREDICTION MODEL BY PERFORMING MACHINE LEARNING
OF CORRESPONDENCE RELATIONSHIP BETWEEN
OBJECTIVE VARIABLE AND EXPLANATORY VARIABLES

END

# FIG.12

PLEASE SELECT RAW MATERIAL CATEGORY TO BE OPTIMIZED (○UNSELECTED, ●SELECTED)

○ALCOHOL RAW MATERIAL    ○ISOCYANATE RAW MATERIAL    ○CROSS-LINKING AGENT    ●CURING AGENT    ○CAT-ALYST

PREDICT

# FIG.13

| NUM-BER | ALCO-HOL 1 | ALCO-HOL 2 | ··· | CROSS-LINKING AGENT 1 | CROSS-LINKING AGENT 2 | ··· | CATA-LYST 1 | CATA-LYST 2 | ··· | FEA-TURE 1 | FEA-TURE 2 | ··· |
|---|---|---|---|---|---|---|---|---|---|---|---|---|
| 1 | | | | | | | | | | | | |
| 2 | | | | | | | | | | | | |
| ⋮ | | | | | | | | | | | | |

MIXING AMOUNTS OF RAW MATERIALS OTHER THAN CURING AGENT      WEIGHTED FEATURES OF CURING AGENT

EP 4 398 257 A1

# FIG.14

START

S30

SET PREDICTION DATA

S32

IDENTIFY DESIGN CONDITION COLUMNS
BASED ON PREDICTION DATA

S34

IDENTIFY COLUMNS OF MAIN RAW MATERIALS AND
COLUMNS OF ADDITIVES BASED ON DESIGN CONDITIONS

S36

CREATE FEATURES WITH RESPECT TO COLUMNS OF ADDITIVES

S38

CALCULATE WEIGHTED FEATURES OF ADDITIVES BASED ON
PRODUCTS OF FEATURES AND MIXING AMOUNTS OF ADDITIVES

S40

CREATE EXPLANATORY VARIABLES BY COMBINING COLUMNS
OF MIXING AMOUNTS OF MAIN RAW MATERIALS WITH
COLUMNS OF WEIGHTED FEATURES OF ADDITIVES

S42

INPUT EXPLANATORY VARIABLES INTO PREDICTION MODEL SO
AS TO PREDICT PROPERTY OF RESIN COMPOSITE MATERIAL

END

## INTERNATIONAL SEARCH REPORT

| International application No. |
| --- |
| **PCT/JP2022/032048** |

| A. | CLASSIFICATION OF SUBJECT MATTER |
| --- | --- |

*G16C 20/70*(2019.01)i
FI: G16C20/70

According to International Patent Classification (IPC) or to both national classification and IPC

| B. | FIELDS SEARCHED |
| --- | --- |

Minimum documentation searched (classification system followed by classification symbols)

G16C20/70

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021/079985 A1 (KONICA MINOLTA INC) 29 April 2021 (2021-04-29) paragraphs [0015]-[0088] | 1-3, 5-11 |
| Y | | 4 |
| Y | JP 2020-77346 A (ASAHI KASEI CORP) 21 May 2020 (2020-05-21) paragraphs [0046]-[0055] | 4 |

☐ Further documents are listed in the continuation of Box C.　　☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **28 October 2022** | **08 November 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/032048**

| Patent document cited in search report | | | Publication date (day/month/year) | Patent family member(s) | Publication date (day/month/year) |
|---|---|---|---|---|---|
| WO | 2021/079985 | A1 | 29 April 2021 | (Family: none) | |
| JP | 2020-77346 | A | 21 May 2020 | CN 111310911 A paragraphs [0072]-[0085] | |

Form PCT/ISA/210 (patent family annex) (January 2015)

**EP 4 398 257 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020077346 A
- JP 6633820 B
- JP 2021140833 A